# EUROPÄISCHE PATENTSCHRIFT

(11) **EP 4 041 412 B1**
(45) Veröffentlichungstag und Bekanntmachung des Hinweises auf die Patenterteilung: **24.04.2024**
(21) Anmeldenummer: 20751130.4
(22) Anmeldetag: 04.08.2020
(51) Int. Cl.: A61Q 19/10, A61K 8/73, A61K 8/46, A61K 8/44, A61K 8/60, C11D 3/382, C11D 1/90, C11D 1/00, C11D 1/66

(54) **REINIGUNGSZUBEREITUNG ENTHALTEND CAESALPINIA SPINOSA GUM**
CLEANSING PREPARATION CONTAINING CAESALPINIA SPINOSA GUM
PRÉPARATION DE NETTOYAGE CONTENANT DE LA GOMME DE CAESALPINIA SPINOSA

(30) Priorität: 11.10.2019 DE 102019215615
(43) Veröffentlichungstag der Anmeldung: 17.08.2022
(73) Patentinhaber: Beiersdorf AG, 22529 Hamburg (DE)
(72) Erfinder: RESCH, Benedict, 22459 Hamburg (DE); GRITZA, Denise, 22529 Hamburg (DE); KOOP, Eefje, 22850 Norderstedt (DE)
(74) Vertreter: Beiersdorf AG
(86) Internationale Anmeldenummer: PCT/EP2020/071870
(87) Internationale Veröffentlichungsnummer: WO 2021/069126

(56) Entgegenhaltungen:
- WO-A1-2019/185915
- DE-A1-102011 084 888
- DATABASE GNPD [Online] MINTEL; 17. Juli 2019 (2019-07-17), anonymous: "Hair & Body Shower Gel", XP055738673, gefunden im www.gnpd.com Database accession no. 6717789
- DATABASE GNPD [Online] MINTEL; 30. Mai 2019 (2019-05-30), anonymous: "Natural Soap No.1", XP055738682, gefunden im www.gnpd.com Database accession no. 6584021
- DATABASE GNPD [Online] MINTEL; 1. März 2019 (2019-03-01), anonymous: "Sport 2 in 1 Hair & Body Wash", XP055738695, gefunden im www.gnpd.com Database accession no. 6373327
- DATABASE GNPD [Online] MINTEL; 22. August 2019 (2019-08-22), anonymous: "Regenerating Body Wash", XP055738742, gefunden im www.gnpd.com Database accession no. 6807057
- Nn: "Tara gum, a safe and natural alternative to guar gum", , 10. Juli 2019 (2019-07-10), Seiten 1-4, XP055738620, Gefunden im Internet: URL:https://web.archive.org/web/2019071002 0718/https://www.silvateam.com/en/products -and-services/food-ingredients/tara-gum/ta ra-gum-safe-and-natural-alternative-guar-g um.html [gefunden am 2020-10-12]
- DATABASE GNPD [Online] MINTEL; 22 August 2019 (2019-08-22), anonymous: "Regenerating Body Wash", XP055738742, Database accession no. 6807057
- DATABASE GNPD [Online] MINTEL; 30 May 2019 (2019-05-30), anonymous: "Natural Soap No.1", XP055738682, Database accession no. 6584021
- DATABASE GNPD [Online] MINTEL; 11 February 2019 (2019-02-11), anonymous: "Tiare Flower Petals Shower Cream", XP055738709, Database accession no. 6328031
- DATABASE GNPD [Online] MINTEL; 17 July 2019 (2019-07-17), anonymous: "Hair & Body Shower Gel", XP055738673, Database accession no. 6717789
- DATABASE GNPD [Online] MINTEL; 1 March 2019 (2019-03-01), anonymous: "Sport 2 in 1 Hair & Body Wash", XP055738695, Database accession no. 6373327

## Beschreibung

Die vorliegende Erfindung betrifft acrylatfreie kosmetische Reinigungszubereitung.

Kosmetische Produkte dienen im Allgemeinen nicht nur dazu schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut eingesetzt.

Die tägliche Körperreinigung mit Duschgelen dient vornehmlich dazu Schweißreste, Fette, abgelagerte Schmutzpartikel und abgestorbene Hautreste effektiv von der Haut zu entfernen. Duschgele enthalten in der Regel anionische Tenside, welche eine besonders effektive Reinigung ermöglichen. Weiterhin enthalten derartige Reinigungsprodukte oftmals zusätzlich weitere Tenside gewählt aus der Gruppe der nichtionischen und amphoteren Tenside.

Neben der reinigenden Wirkung stehen weitere Produktmerkmale im Vordergrund, die für die Anwendung durch den Verbraucher relevant sind. So sollten Reinigungszubereitungen zur Anwendung unter der Dusche folgende Parameter erfüllen:
Sensorische Eigenschaften beim Verteilen auf nasser Haut:
   - Starke Glitschigkeit des Produktes an sich, wenn das Produkt zwischen Fingern und der aufzutragenden Körperfläche verrieben wird
   - Keine Fadenziehung beim Verteilen
   - Geringer Kraftaufwand beim Verteilen auf der Haut, bzw. leichte Anhaftung des Produkts an die Haut
   - Gleichmäßige Verteilbarkeit beim Auftragen
Sensorische Eigenschaften während und nach dem Aufschäumen:
   - Die Ausbildung eines voluminösen Schaums
   - Die Ausbildung großer Luftblasen beim Aufschäumen
Sensorische Eigenschaften beim oder nach dem Abspülen mit Wasser:
   - Schnelle und leichte Abspülbarkeit mit Wasser nach dem Auftragen
   - Keine klebrigen Rückstände nach dem Abspülen mit Wasser
   - Glattes Hautgefühl, wenn mit der Hand über die mit Wasser abgespülte Hautfläche gestrichen wird.

Derartige sensorische Eigenschaften lassen sich üblicher Weise am besten mittels Probandenstudien evaluieren, bei denen anhand eines standardisierten Protokolls die Produkteigenschaften evaluiert werden.

Die sensorischen Eigenschaften beim Verteilen lassen sich analysieren, indem ein ausgewähltes Hautareal (bspw. Pulshöhe) zunächst mit Wasser gereinigt wird, auf die genässte Haut 0,5 ml des Reinigungsprodukts appliziert wird, das Areal nochmal befeuchtet wird und abschließend mit einem Finger in kreisenden Bewegungen das Reinigungsprodukt verteilt wird. Bei diesem Verfahren dient die erste kreisende Bewegung zunächst dazu einen Produktfilm zu erzeugen. Sind zur Filmerzeugung mehrere Kreisbewegungen nötig, liegt eine schlechtere Verteilbarkeit vor. Gleichzeitig, wird beurteilt, in wie weit Kraft aufgewendet werden muss, um einen Produktfilm zu erzeugen. Sobald sich ein erster Film gebildet hat, ist es möglich mit den nächsten Kreisbewegungen mit dem Finger die Glitschigkeit des Produktes an sich zu beurteilen, da nun das Produkt besonders leicht über den zuvor gebildeten Produktfilm gleiten sollte. Somit bezieht sich der Ausdruck "Giitschigkeit" auf die Gleiteigenschaften des Produkts auf einem auf der Haut vorliegenden Produktfilm. Ein weiterer Aspekt, der beim kreisförmigen Verteilen des Produkts evaluiert wird, ist in wie weit das Produkt beim Verteilen Fäden zieht.

Die sensorischen Eigenschaften des Reinigungsprodukts beim Aufschäumen lassen sich analysieren, indem auf einem mit Wasser befeuchteten Testareal auf dem Unterarm eine festgelegt Menge des Produkts aufgetragen wird. Anschließend wird mit einer nassen Handinnenfläche mehrfach über das Areal gestrichen und so das applizierte Produkt aufgeschäumt. Nach einer festgelegten Anzahl an Bewegungen wird der gebildete Schaum beurteilt, wobei das gesamte Schaumvolumen und die Blasengröße die zu bestimmenden Parameter darstellen.

Die sensorische Eigenschaften beim oder nach dem Abspülen mit Wasser lassen sich analysieren, indem das Hautareal mit dem zuvor aufgeschäumte Produkt horizontal unter Wasser gehalten wird und wobei im vorgegebenen Takt der Schaum mit der Handinnenfläche abgespült wird. Während dieses Vorgangs lässt sich beurteilen, wie schnell sich das Schaum abspülen lässt. Gleichzeitig stellt das Maß der Reibung ausgeübt durch die Handinnenfläche eine Möglichkeit zur Beurteilung dar, wie leicht das Produkt abgespült werden kann. Nach dem Abspülen können die Klebrigkeit der Rückstände sowie die Hautglätte mittels Streichen mit der Hand über das gereinigte Areal bestimmt werden. Konventionelle Reinigungszubereitungen zur Hautreinigung unter der Dusche sind unter anderem aus EP1647591 A1 weitreichend bekannt. Die Reinigungszubereitungen enthalten üblicher Weise anionische und amphotere Tenside, ein verdickendes Polymer auf Basis von Homo- oder Copolymeren von Acrylsäure und üblicher Weise mindestens zusätzlich ein Mittel, welches neben weiteren Aspekten die Glitschigkeit der Zubereitung nach erster Filmbildung auf der Haut erhöht.

Jedoch bevorzugen Konsumenten zunehmend Reinigungszubereitungen, welche keine Homo- oder Copolymere von Acrylsäure enthalten, da deren Umweltabbaubarkeit derzeit diskutiert wird. Problematisch ist dabei der Umstand, dass Reinigungszubereitungen zur Anwendung unter der Dusche ohne Homo- oder Copolymere von Acrylsäure oftmals nicht die vom Verbraucher gewünschte Glitschigkeit beim Auftragen auf nasse Haut zeigen. Es sind Reinigungszubereigungen bekannt, welche Caesalpinia Spinosa Gum enthalten (Mintel GNPD "Hair & Body Shower Gel", XP055738673, Database accession no.6717789; "Natural Soap No.1", XP055738682, Database accession no.6584021; "Sport 2-in-1 Hair & Body Wash", XP055738695, Database accession no.6373327).

Folglich war es Aufgabe der vorliegenden Erfindung, Reinigungszubereitungen bereitzustellen, welche ohne den Einsatz von Homo und Copolymeren von Acrylsäure eine erhöhte Glitschigkeit aufweisen, wenn die Reinigungszubereitung auf nasse Haut aufgetragen wird und sich ein erster Film aus Bestandteilen der Reinigungszubereitung auf der Haut gebildet hat. Entsprechenden soll sich die Reinigungszubereitung beim Verteilen auf der Haut bzw. auf dem auf der Haut durch die Reinigungszubereitung gebildeten Film besonders glitschig anfühlen.

Überraschend wurde nun gefunden, dass die Anforderungen durch die vorliegende Erfindung gelöst werden konnten.

Gegenstand der Erfindung ist eine kosmetische Reinigungszubereitung zur Anwendung unter der Dusche umfassend, bezogen auf das Gesamtgewicht der Zubereitung,
a) 0,5 bis 8 Gew.-% anionische und/oder nichtionische Tenside,
b) 2,5 bis 7 Gew.-% amphotere und/oder zwitterionische Tenside, und
c) Caesalpinia Spinosa Gum,
dadurch gekennzeichnet, dass keine Homo- oder Copolymere von Acrylsäure enthalten sind und dass der Anteil von Caesalpinia Spinosa Gum von 0,1 bis 1 Gew.-% beträgt.

Sollten nachfolgend Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der kosmetischen

Reinigungszubereitung. Sollten nachfolgend Verhältnisse von Komponenten/Substanzen/Stoffgruppen offenbart werden, so beziehen diese Verhältnisse auf Gewichtsverhältnisse der genannten Komponenten/Substanzen/Stoffgruppen.

Die Formulierungen "erfindungsgemäß", "erfindungsgemäß vorteilhaft", "vorteilhaft im Sinne der Vorliegenden Erfindung" etc. beziehen sich im Rahmen der vorliegenden Offenbarung immer sowohl auf die erfindungsgemäße Zubereitung sowie die erfindungsgemäße Verwendung und das erfindungsgemäße Verfahren.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Anionische Tenside weisen als funktionelle Gruppen in der Regel Carboxylat-, Phosphat-, Sulfat- oder Sulfonatgruppen auf. In wässriger Lösung bilden sie im sauren oder neutralen Milieu negativ geladene organische Ionen.

Erfindungsgemäß vorteilhaft einzusetzende anionische Tenside sind Schwefelsäureester, wie
- Alkylethersulfat, insbesondere Natrium-, Ammonium-, Magnesium-, MIPA-, TIPA-Laurylethersulfat, Natriummyristylethersulfat und die unter der INCI Bezeichnung bekannte Substanz Sodium C12-13-Parethsulfat;
- Alkylsulfate, insbesondere Natrium-, Ammonium- und TEA-Laurylsulfat;

sowie Sulfonsäuren und Salze, wie
   - Acyl-isethionate, insbesondere Natrium-/ Ammoniumcocoyl-isethionat;
   - Alkylarylsulfonate;
   - Alkylsulfonate, insbesondere Natriumcocosmonoglyceridsulfat, Natrium C12-14 Olefinsulfonat, Natriumlaurylsulfoacetat und Magnesium PEG-3 Cocamidsulfat,
   - Sulfosuccinate, insbesondere Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat;
Weitere vorteilhaft einzusetzende anionische Tenside sind Acylaminosäuren und deren Salze, wie
   - Acylglutamate, insbesondere Natriumacylglutamat, Di-TEA-palmitoylaspartat und Natrium Caprylic/ Capric Glutamat;
   - Acylpeptide, insbesondere Palmitoyl-hydrolysiertes Milchprotein, Natrium Cocoyl-hydrolysiertes Soja Protein und Natrium-/ Kalium-Cocoyl-hydrolysiertes Kollagen,
   - Acylsarcosinate, insbesondere Myristoyl Sarcosin, TEA-lauroyl Sarcosinat, Natriumlauroylsarcosinat und Natriumcocoylsarkosinat;
   - Acyltaurate, insbesondere Natriumlauroyltaurat und Natriummethylcocoyltaurat;
   - Acyllactylate, insbesondere Lauroyllactylat, Caproyllactylat;
   - Acylalaninate;
   - Acylglycinate, insbesondere Natriumcocoylglycinat.

Weitere vorteilhaft einzusetzende anionische Tenside sind die nachfolgend spezifizierten Gruppen der Carbonsäuren und Carbonsäurederivaten:
- Carbonsäuren, insbesondere Laurinsäure, Aluminiumstearat, Magnesiumalkanolat und Zinkundecylenat;
- Ester-Carbonsäuren, beispielsweise Calciumstearoyllactylat, Laureth-6-Citrat und Natrium PEG-4-Lauramidcarboxylat;
- Ether-Carbonsäuren, beispielsweise Natriumlaureth-13-Carboxylat und Natrium PEG-6-Cocamide Carboxylat;
sowie Phosphorsäureester und Salze, wie beispielsweise DEA-Oleth-10-Phosphat und Dilaureth-4 Phosphat.

Es ist insbesondere vorteilhaft, wenn die erfindungsgemäße kosmetische Reinigungszubereitung ein oder mehrere anionische Tenside gemäß der Formel (I)

RO-(CH₂CH₂O)ₓ-(CH₂-CHR¹O)_{y}-(CH₂CH₂O)_{z}-SO₃⁻ M⁺ (I)

enthält, dadurch gekennzeichnet, dass
R ein linearer oder verzweigter C₈-C₁₈-Alkylrest oder Mischungen verschiedener linearer oder verzweigter C₈-C₁₈-Alkylreste ist;
R¹ ein Methyl, Ethyl oder Gemische davon ist;
M⁺ ein Kation, ausgewählt aus der Gruppe bestehend aus Alkalimetallen, NH₄⁺ und HNR² ₃⁺ ist, wobei R² ausgewählt ist aus der Gruppe bestehend aus linearen oder verzweigten Alkylresten, CH₂CH₂OH und CH₂CH(OH)CH₃;
x eine ganzzahlige Zahl im Bereich von 0 - 3 ist;
y eine ganzzahlige Zahl im Bereich 0 - 10 ist; und
z eine ganzzahlige Zahl im Bereich 0 - 30 ist.

Besonders bevorzugte Tenside der Formel (I) sind gewählt aus der Gruppe Sodium Laureth Sulfate (Natriumlaurylethersulfat) und/oder Ammonium Laureth Sulfate (Ammoniumlaurylethersulfat). Enthält die erfindungsgemäße kosmetische Zubereitung ein anionisches Tensid gemäß der Formel (I), vorzugsweise Sodium Laureth Sulfate (Natriumlaurylethersulfat) und/oder Ammonium Laureth Sulfate (Ammoniumlaurylethersulfat), so ist es bevorzugt, wenn der Anteil dieser anionischen Tenside von 0,5 Gew.-% bis 8 Gew.-% bevorzugt von 2 Gew.-% bis 7,5 Gew.-% und insbesondere bevorzugt von 4 Gew.-% bis 7 Gew.-% bezogen auf das Gesamtgewicht der kosmetischen Zubereitung beträgt.

Insbesondere vorteilhaft sind Sodium Laureth Sulfate (Natriumlaurylethersulfat) und/oder Ammonium Laureth Sulfate in einem Anteil von 0,5 Gew.-% bis 8 Gew.-% bevorzugt von 2 Gew.-% bis 7,5 Gew.-% und insbesondere bevorzugt von 4 Gew.-% bis 7 Gew.-% bezogen auf das Gesamtgewicht der kosmetischen Zubereitung enthalten. Dabei ist es insbesondere orteilhaft, wenn neben Sodium Laureth Sulfate keine weiteren anionischen Tenside enthalten sind. Ausführungsformen, welche eine besonders effektive Reinigungsleistung liefern sollen, sind dabei vorteilhaft dadurch gekennzeichnet, dass anionische Tenside enthalten sind und auf die nicht ionischen Tenside verzichtet wird.

Sofern nichtionische Tenside enthalten sind, sind diese vorteilhaft gewählt aus Gruppe:
- Fettsäurealkanolamide der allgemeinen Formel (II) 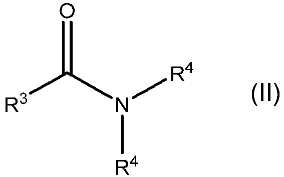 wobei *R³* ein linearer oder verzweigter, gesättigter oder ungesättigter Alkyl- oder Alkenylrest mit 8 bis 24 Kohlenstoffatomen ist, *R⁴* je für ein Wasserstoffatom oder eine -(CH₂)ₙOH Gruppe stehen, in der *n* die Zahlen 2 oder 3 annehmen kann, mit der Maßgabe, dass mindestens eine der Gruppen *R⁴* eine -(CH₂)ₙOH Gruppe ist, beispielsweise Cocamide MEA/ DEA /MIPA;
- Anlagerungsprodukte von Ethylenoxid an Fettsäurealkanolamide
- C₈-C₃₀-Fettsäuremono- und/oder diester von Anlagerungsprodukten von 1 bis 50 mol Ethylenoxid an Glycerin;
- Anlagerungsprodukte von 2 bis 50 mol Ethylenoxid und/oder 0 bis 5 mol Propylenoxid an lineare oder verzweigte Fettalkohole mit 8 bis 30 Kohlenstoffatomen, an Fettsäuren mit 8 bis 30 Kohlenstoffatomen und an Alkylphenole mit 8 bis 15 Kohlenstoffatomen in der Alkylgruppe; und
- Alkylpolyglycoside, wie bevorzugt Laurylglycosid, Decylglycosid und Cocoglycosid.

Vorteilhafte Ausführungsformen der Erfindung sind dadurch gekennzeichnet, dass diese als nichtionische Tenside Alkylglycoside, im speziellen Decylglycosid und/oder Cocoglycosid, enthalten. Enthält die erfindungsgemäße kosmetische Reinigungszubereitung Alkylglycoside, im speziellen Decylglycosid und/oder Cocoglycosid, so ist es bevorzugt, wenn der Anteil der Alkylglycoside von 0,5 Gew.-% bis 5 Gew.-% und bevorzugt von 2 Gew.-% bis 4 Gew.-%, bezogen auf das Gesamtgewicht der Reinigungszubereitung, beträgt.

Durch den Einsatz der erfindungsgemäßen nichtionischen Tenside, insbesondere durch den Einsatz der nichtionischen Tenside in einem Gesamtanteil von 0,1 Gew.-% bis 5 Gew.-%, bezogen auf das Gesamtgewicht der Reinigungszubereitung, wird die Schaumbildung der Reinigungszubereitung gefördert. Zudem ermöglicht ein derartiger Einsatz von nichtionischen Tensiden die Viskosität der Reinigungszubereitung zu erhöhen. Innerhalb dieser Ausführungsformen ist es weiterhin bevorzugt, wenn keine anionischen Tenside enthalten sind. Entsprechend sind die Reinigungszubereitungen besonders milde zur Haut. Ferner enthält die erfindungsgemäße kosmetische Reinigungszubereitung ein oder mehrere amphotere und/oder zwitterionische Tenside. Vorteilhaft zu verwendende amphotere und/oder zwitterionische Tenside sind
- Acyl-/dialkylethylendiamin, insbesondere Natriumacylamphoacetat, Dinatriumacylamphodipropionat, Dinatriumalkylamphodiacetat, Dinatrium Cocoamphodiacetat, Natrium Cocoamphomonoacetat, Natriumacylamphohydroxypropylsulfonat, Dinatriumacylamphodiacetat und Natriumacylamphopropionat;
- N-Alkylaminosäuren, insbesondere Aminopropylalkylglutamid, Alkylaminopropionsäure, Natriumalkylimidodipropionat und Lauroamphocarboxyglycinat;
- Betaine, insbesondere Coco Betaine, Cocoamidopropyl Betaine; und
- Sultaine, insbesondere Lauryl Hydroxy Sultaine.

Insbesondere vorteilhaft sind die unter den INCI-Bezeichnungen bekannten amphotere und/oder zwitterionische Tenside Sodium Cocoamphoacetate, Disodium Cocoamphodiacetate, Sodium Cocoamphopropionate, Disodium Cocoamphodipropionate, Coco Betaine, Lauryl Betaine und/oder Cocamidopropylbetain enthalten. Aus der vorstehenden Gruppe wird Cocamidopropylbetain als bevorzugtestes amphoteres und/oder zwitterionisches Tensid gewählt.

Der Anteil der amphoteren und/oder zwitterionischen Tenside, insbesondere der zuvor als insbesondere vorteilhaft gelisteten amphoteren und/oder zwitterionischen Tenside, in der erfindungsgemäßen kosmetischen Reinigungszubereitung beträgt 2,5 bis 7 Gew.-%, bevorzugt von 3 Gew.-% bis 6 Gew.-% und insbesondere bevorzugt von 3,5 Gew.-% bis 5,5 Gew.-%, bezogen auf das Gesamtgewicht der Reinigungszubereitung.

Vorteilhafte Ausführungsformen der Erfindung sind daher dadurch gekennzeichnet, dass Cocamidopropylbetain in der erfindungsgemäßen kosmetischen Reinigungszubereitung in einem Anteil von 2,5 bis 7 Gew.-%, bevorzugt von 3 Gew.-% bis 6 Gew.-% und insbesondere bevorzugt von 3,5 Gew.-% bis 5,5 Gew.-%, bezogen auf das Gesamtgewicht der Reinigungszubereitung, enthalten ist. Innerhalb dieser Ausführungsformen ist es weiterhin insbesondere bevorzugt, wenn neben Cocamidopropylbetain keine weiteren amphoteren und/oder zwitterionischen Tenside enthalten sind.

Erfindungsgemäß umfasst die kosmetische Reinigungszubereitung Caesalpinia Spinosa Gum. Der Anteil von Caesalpinia Spinosa Gum beträgt von 0,1 bis 1 Gew.-%, bevorzugt von 0,15 bis 0,46 Gew.-% und insbesondere bevorzugt von 0,2 bis 0,4 Gew.-%, bezogen auf das Gesamtgewicht der Zubereitung.

Es war für den Fachmann überaus überraschend, dass bereits bei einer Zugabe von geringen Anteilen an Caesalpinia Spinosa Gum von bis zu 0,4 Gew.-% sich bereits eine deutliche Steigerung in Bezug auf die Glitschigkeit, wie vorstehend beschrieben, erzeugen lässt. Andere Polymere, welche zu einer Zunahme der Glitischigkeit eingesetzt werden könnten, benötigen zumeist höhere Einsatzkonzentrationen. Folglich ergibt sich zumeist eine deutlich erhöhte Viskosität für die Zubereitung. Überraschend konnte jedoch nun gefunden werden, dass durch Einsatz der Erfindung Zubereitungen mit geringerer Viskosität und hoher Glitschigkeit, wie vorstehen beschrieben, erhalten werden können. Somit sind die Zubereitungen deutlich flexibler einsetzbar, da auch weniger Viskose Reinigungszubereitungen für die Dusche erhalten werden. So ist eine Entnahme über einen Pumpspender beispielsweise möglich.

Gemäß der vorliegenden Erfindung ist es in manchen Ausführungsformen vorteilhaft, wenn neben Caesalpinia Spinosa Gum keine weiteren natürlichen polymeren Verdicker enthalten sind.

Andere vorteilhafte Ausführungsformen der Erfindung sind dadurch gekennzeichnet, dass diese zusätzlich Xanthan Gum zur Einstellung der Viskosität der Zubereitung enthalten. Ist Xanthan Gum enthalten beträgt der Gesamtanteil von Xanthan Gum bevorzugt von 0,01 bis 0,3 Gew.-% bezogen auf das Gesamtgewicht der Reinigungszubereitung.

Caesalpinia Spinosa Gum ist ein nichtionisches Polysaccharide, was in Pulverform vorliegt. Kommerziell kann es von der Fa. Seppic unter dem Handelsnamen SOLAGUM^{™} TARA bezogen werden.

Weiterhin ist es vorteilhaft im Sinne der vorliegenden Erfindung, wenn die Zubereitung PEG-7 Glyceryl Cocoate enthält.

Der Gesamtanteil von PEG-7 Glyceryl Cocoate in der kosmetischen Reinigungszubereitung ist vorteilhaft gewählt von 0,2 Gew.-% bis 5 Gew.-%, bevorzugt von 0,5 Gew.-% bis 2,5 Gew.-% und insbesondere bevorzugt von 1 Gew.-% bis 2 Gew.-%, bezogen auf das Gesamtgewicht der Reinigungszubereitung.

Ferner ist es erfindungsgemäß vorteilhaft, wenn zusätzlich PEG-200 Hydrogenated Glyceryl Palmate, PEG-40 Hydrogenated Castor Oil und/oder PEG-90 Glyceryl Isostearate enthalten sind. Dabei ist es vorteilhaft, wenn der Anteil von PEG-200 Hydrogenated Glyceryl Palmate, PEG-40 Hydrogenated Castor Oil und/oder PEG-90 Glyceryl Isostearate von 0,1 Gew.-% bis 1,5 Gew.-%, bevorzugt von 0,2 Gew.-% bis 1,3 Gew.-%, insbesondere bevorzugt von 0,5 Gew.-% bis 1 Gew.-%, bezogen auf das Gesamtgewicht der Reinigungszubereitung, beträgt. Es ist insbesondere vorteilhaft, wenn mindestens PEG-40 Hydrogenated Castor Oil in einem Anteil von 0,1 Gew.-% bis 1,5 Gew.-%, bevorzugt von 0,2 Gew.-% bis 1,3 Gew.-%, insbesondere bevorzugt von 0,5 Gew.-% bis 1 Gew.-%, bezogen auf das Gesamtgewicht der Reinigungszubereitung, enthalten ist.

Weiterhin ist es vorteilhaft, wenn die kosmetischen Reinigungszubereitungen gemäß der vorliegenden Erfindung Wasser als kosmetischen Träger enthält, wobei Wasser in einem Anteil von 70 Gew.-% bis 92 Gew.-%, bevorzugt von 75 Gew.-% bis 90 Gew.-% und insbesondere bevorzugt von 80 Gew.-% bis 88 Gew.-% bezogen auf das Gesamtgewicht der Reinigungszubereitung enthalten ist.

Ferner ist es vorteilhaft im Sinne der vorliegenden Erfindung, wenn die Zubereitung frei von Silikonölen ist.

Zur Konservierung der vorliegenden kosmetischen Reinigungszubereitungen können die in der Kosmetik üblicherweise verwendeten Konservierungsmittel eingesetzt werden. Dazu zählen beispielsweise Parabene, wie Methylparaben, Propylparaben, Ethylparaben und Butylparaben. Wünschenswert ist aber auch der Einsatz von Konservierungsmitteln auf Säure-Basis, die in der Nahrungsmittelindustrie zum Einsatz kommen. Hierzu zählen beispielsweise Benzoesäure und/oder Salicylsäure und/oder deren Salze. Es ist insbesondere bevorzugt, wenn Natriumbenzoat enthalten ist, wobei der Anteil von Natriumbenzoat bevorzugt im Bereich von 0,2 bis 0,65 Gew.-% bezogen auf das Gesamtgewicht der Zubereitung beträgt.

Weiterhin hat es sich als vorteilhaft herausgestellt, wenn die Zubereitung mindestens Zitronensäure oder deren Salze umfasst. Ist Zitronensäure oder deren Salze enthalten, so ist es vorteilhaft wenn der Anteil dieser Komponenten von 0,2 bis 1 Gew.-% bezogen auf das Gesamtgewicht der Reinigungszubereitung beträgt.

Vorteilhaft ist die Reinigungszubereitung der Erfindung dadurch gekennzeichnet, dass diese unter Normalbedingungen eine Viskosität von weniger als 10000 mPa·s, weiterhin bevorzugt weniger als 5000 mPa·s, weiterhin bevorzugt von weniger als 4000 mPa·s und insbesondere bevorzugt weniger als 3500 mPa·s auf. Weiterhin ist es vorteilhaft, wenn die kosmetische Zubereitung eine Viskosität von mindestens 500 mPa s, bevorzugt mindestens 1500 mPa s, weiterhin bevorzugt mindestens 2000 mPa·s und insbesondere bevorzugt mindestens 2300 mPa·s aufweist. Entsprechend beträgt die Viskosität der kosmetischen Zubereitung vorteilhaft 500 bis 10000 mPa s, bevorzugt 1500 bis 5000 mPa s, weiterhin bevorzugt 2000 bis 4000 mPa·s und insbesondere bevorzugt 2300 mPa·s bis 3500 mPa·s.

Werden in dieser Offenbarung Viskositätswerte angegeben, so beziehen sich alle Werte auf eine Messung bei 25°C in einer 150 ml Weithalsflasche (VWR Nr.: 807-001) mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messsystems wird die Viskosität berechnet. Als Messkörper wird der Messkörper Nr.1 (Artikelnr. 200 0191), geeignet für einen Viskositätsbereich bis 10.000 [mPa s], Drehzahl Bereich 62,5 min⁻¹, verwendet. Erfolgt keine andere Angabe, so erfolgt die Messung zur Viskosität immer 24h nach Herstellung der Zubereitung.

Weiterhin sind vorteilhafte Ausführungsformen der Erfindung dadurch gekennzeichnet, dass der Gesamtanteil aller enthaltenen Tenside maximal 14 Gew.-%, bevorzugt maximal 13,5 Gew.-% und insbesondere maximal 12,5 Gew.-% beträgt, wobei sich die Angaben auf das Gesamtgewicht der kosmetischen Reinigungszubereitung beziehen.

Weiterhin enthält die erfindungsgemäße kosmetische Reinigungszubereitung vorteilhaft mindestens ein natürliches Öl. Unter natürlichen Ölen werden alle Öle von pflanzlichen und tierischen Ursprungs verstanden. Insbesondere bevorzugt werden ausschließlich Öle pflanzlichen Ursprungs als natürliche Öle eingesetzt.

Zu den erfindungsgemäß vorteilhaft sind die natürlichen Öle gewählt aus der Gruppe Sonnenblumenöl (Helianthus Annuus Seed Oil), Rapsöl (Canola Oil), Sojaöl (Glycine Soja Oil), Olivenöl (Olea Europaea Fruit Oil), Mandelöl (Prunus amygdalus dulcis Oil), Avocadoöl (Persea Gratissima Oil), Walnussöl (Junglans Regia Seed Oil), Pfirsichkernöl (Prunus Persica Kernel Oil), Aprikosenkernöl (Prunus Armeniaca Kernel Oil), Sesamöl (Sesamum Indicum Seed Oil), Camelienöl (Camelia Oleifera/ Camelia Sasanqua), Nachtkerzenöl (Oenothera biennis), Macadamianussöl (Macadamia Intergrifolia Seed Oil), Diestelöl (Silybum Marianum Seed Oil), Weizenkeimöl (Triticum Vulgare Germ Oil), Palmkernöl (Elaeis guineensis Kernel Oil), Palmöl (Elaeis guineensis Oil), Traubenkernöl (Vitis Vinifera Seed Oil), Arganöl (Argania spinosa Seed Oil), Erdnussöl (Arachis hypogaea Oil), Kürbiskernöl (Cucurbita Pepo Seed Oil), Ricinusöl (Ricinus Communis Seed Oil), Reiskeimöl (Oryza Sativa Bran Oil), Vegetable Oil (Olus Oil) und Mischungen dieser Öle.

Der Einsatz von pflanzlichen Ölen ist gegenüber den tierischen Ölen bevorzugt, wobei insbesondere die natürlichen Öle gewählt aus der Gruppe Sojaöl (Glycine Soja Oil), Sonnenblumenöl (Helianthus Annuus Seed Oil), Rapsöl (Canola Oil) und Olivenöl (Olea Europaea Fruit Oil) in der kosmetischen Reinigungszubereitung enthalten sind.

Weiterhin ist es bevorzugt, wenn die natürlichen Öle vorteilhaft in einem Anteil von mindestens 0,05 Gew.-%, bevorzugt mindestens 0,1 Gew.-%, weiterhin bevorzugt mindestens 0,2 Gew.-%, weiterhin bevorzugt mindestens 0,3 Gew.-%, weiterhin bevorzugt mindestens 0,4 Gew.-%, weiterhin bevorzugt mindestens 0,5 Gew.-%, weiterhin bevorzugt mindestens 0,6 Gew.-% und insbesondere bevorzugt mindestens 0,7 Gew.-% bezogen auf das Gesamtgewicht der Zubereitung enthalten sind.

Erfindungsgemäß vorteilhaft umfasst die Reinigungszubereitung vorteilhaft Natriumchlorid. Ist Natriumchlorid enthalten, so beträgt der Anteil von Natriumchlorid vorteilhaft von 0,15 bis 1,2 Gew.-% und insbesondere vorteilhaft von 0,6 bis 1 Gew.-% bezogen auf das Gesamtgewicht der Zubereitung.

Die erfindungsgemäßen Reinigungszubereitungen können vorteilhaft Opacifier enthalten. Opacifier sind in Wasser nicht lösliche, meist mikropartikuläre Substanzen, die das Licht besonders gut reflektieren und der Reinigungszubereitungen eine milchige oder eingetrübte Erscheinung verleihen. Erfindungsgemäße Opacifier sind Glycol Distearate, Glyceryl Stearate oder PEG-3 Distearate.

Ferner können die erfindungsgemäßen Reinigungszubereitungen weitere Substanzen gewählt aus der Gruppe Glycerin, Propandiol und Sorbitol enthalten.

Die kosmetische Zubereitung gemäß der Erfindung können ferner weitere kosmetische Hilfsstoffe und Wirkstoffe enthalten, wie sie üblicherweise in solchen Zubereitungen verwendet werden, z. B. weitere Wirkstoffe, Konservierungsmittel, Konservierungshelfer, Bakterizide, Farbstoffe und Farbpigmente, Verdickungsmittel, anfeuchtende und/oder feuchthaltende Substanzen oder andere übliche Bestandteile einer kosmetischen oder dermatologischen Formulierung wie weitere Polyole, Schaumstabilisatoren, organische Lösungsmittel oder Silikonderivate, sofern der Zusatz die geforderten Eigenschaften hinsichtlich der Stabilität nicht beeinträchtigen oder ausgeschlossen sind.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Es wurden die folgenden Formulierungen hergestellt. Die Rezepturen Vgl.1 und Vgl.2 sind nicht erfindungsgemäße, während Bsp.1 erfindungsgemäß ist:

| **Inhaltsstoffe** | **Vgl.1** | **Vgl.2** | **Bsp.1** |
|---|---|---|---|
| Sodium Laureth Sulfate | 6,51 | 6,51 | 6,51 |
| Cocamidopropyl Betaine | 4,51 | 4,51 | 4,51 |
| Glycerin | 0,4 | 0,4 | 0,4 |
| Natriumchlorid | 0,8 | 0,8 | 0,8 |
| PEG-40 Hydrogenated Castor Oil | 0,5 | 0,5 | 0,5 |
| PEG-7 Glyceryl Cocoate | 1,5 | 1,5 | 1,5 |
| Citric Acid | 0,55 | 0,55 | 0,55 |
| Sodium Benzoate | 0,45 | 0,45 | 0,45 |
| Parfum | 0,9 | 0,9 | 0,9 |
| Ceratonia Siliqua (Carob) Gum¹ | | 0,25 | |
| Caesalpinia Spinosa Gum² | | | 0,25 |
| Aqua | ad 100 | ad 100 | ad 100 |
| Viskosität in mPa·s³ | 1850 | 2550 | 2700 |

| | | | |
|---|---|---|---|
| ¹Handelsprodukt Genu Gum RL-200Z-CG von der Fa. Rahn ²Handelsprodukt SOLAGUM^{™} TARA von der Fa. Seppic ³Messverfahren siehe Beschreibung. | | | |

Die vorstehend aufgeführten Reinigungszubereitungen wurden hergestellt und deren Eigenschaften in einem Experten-Waschpanel untersucht. Die folgende Prozedur wurde angewendet:

### Vorbereitung:

Die Unterarme der Teilnehmer wurden mit einem Standardshampoo gereinigt, abgetrocknet und für 10 Minuten akklimatisiert. Anschließend wurde die innere Seite des Unterarms für 2 Sekunden unter fließendes Wasser gehalten und 0,5 ml der zu untersuchenden Zubereitung auf einen kreisförmigen Bereich mit 3 cm Durchmesser auf dem inneren Unterarm appliziert. Vor dem Verteilen wurde die verteilende Hand 2 Sekunden unter fließendes Wasser gehalten. Dann wurden die Finger gespreizt um Wasser abtropfen zu lassen. Die Zubereitung wurde dann mit 2 Fingern der verteilenden Hand in kreisförmigen Bewegungen (5 cm Durchmesser) mit 90 bpm (beat per minute) verteilt, wobei nach 5 Kreisen die Glitschigkeit beurteilt wird. Als Skalar wird ein Wertebereich von 1 bis 5 vorgegeben, wobei 1 nicht Glitschig und 5 stark Glitschig bedeutet.

Jede Zubereitung wurde 6 Mal von verschiedenen Experten analysiert. In der nachfolgenden Tabelle sind die erhaltenen Mittelwerte für die Glitschigkeit aufgeführt:

| **Zubereitung** | **Glitschigkeit Messwert (Mittelwert)** |
|---|---|
| **Vgl.1** | 1,8 |
| **Vgl.2** | 2,8 |
| **Bsp.1** | 3,5 |

Folglich wurde gefunden, dass die erfindungsgemäße Reinigungszubereitung eine deutlich erhöhte Glitschigkeit beim Verteilen auf nasser Haut, auf der sich ein Produktfilm gebildet hat, aufweist.

| **Inhaltsstoffe** | **Bsp.2** | **Bsp.3** | **Bsp.4** | **Bsp.5** | **Bsp. 6** |
|---|---|---|---|---|---|
| Sodium Laureth Sulfate | 6,3 | 6,3 | 3,15 | | |
| Cocamidopropyl Betaine | 4,5 | 3,0 | 3,5 | 5,0 | 3,0 |
| Glycerin | 0,135 | 0,09 | 0,105 | 0,15 | 0,09 |
| Natriumchlorid | 0,27 | 0,18 | 0,21 | 0,3 | 0,18 |
| Coco Glucoside | | | | 2,6 | 1,56 |
| Decyl Glucoside | | | | | 1,59 |
| PEG-40 Hydrogenated Castor Oil | 0,5 | 0,5 | 0,5 | 0,3 | 0,3 |
| Parfum | 1 | 1 | 1 | 0,5 | 0,5 |
| Sodium Benzoate | 0,5 | 0,5 | 0,55 | 0,45 | 0,5 |
| Citric Acid | 0,5 | 0,5 | 0,5 | 0,5 | 0,5 |
| Caesalpinia Spinosa Gum | 0,25 | 0,1 | 0,5 | 0,3 | 0,45 |
| Xanthan Gum | | 0,2 | | 0,1 | |
| Helianthuus Annus Seed Oil | 0,05 | 0,1 | | | |
| Aqua | ad 100 | ad 100 | ad 100 | ad 100 | ad 100 |

## Patentansprüche

1. Kosmetische Reinigungszubereitung zur Anwendung unter der Dusche umfassend, bezogen auf das Gesamtgewicht der Zubereitung,
a) 0,5 bis 8 Gew.-% anionische und/oder nichtionische Tenside,
b) 2,5 bis 7 Gew.-% amphotere und/oder zwitterionische Tenside, und
c) Caesalpinia Spinosa Gum,
**dadurch gekennzeichnet, dass** keine Homo- oder Copolymere von Acrylsäure enthalten sind und dass der Anteil von Caesalpinia Spinosa Gum von 0,1 bis 1 Gew.-% beträgt.

2. Reinigungszubereitung nach Anspruch 1 **dadurch gekennzeichnet, dass** ein oder mehrere anionische Tenside gemäß der Formel (I)
RO-(CH₂CH₂O)ₓ-(CH₂-CHR¹O)_{y}-(CH₂CH₂O)_{z}-SO₃⁻ M⁺ (I)
enthalten sind, **dadurch gekennzeichnet, dass**
R ein linearer oder verzweigter C₈-C₁₈-Alkylrest oder Mischungen verschiedener linearer oder verzweigter C₈-C₁₈-Alkylreste ist;
R¹ ein Methyl, Ethyl oder Gemische davon ist;
M⁺ ein Kation, ausgewählt aus der Gruppe bestehend aus Alkalimetallen, NH₄⁺ und HNR² ₃⁺ ist, wobei R² ausgewählt ist aus der Gruppe bestehend aus linearen oder verzweigten Alkylresten, CH₂CH₂OH und CH₂CH(OH)CH₃;
x eine ganzzahlige Zahl im Bereich von 0 - 3 ist;
y eine ganzzahlige Zahl im Bereich 0 - 10 ist; und
z eine ganzzahlige Zahl im Bereich 0 - 30 ist.

3. Reinigungszubereitung nach einem der vorhergehenden Ansprüche **dadurch gekennzeichnet, dass** Sodium Laureth Sulfate und/oder Ammonium Laureth Sulfate enthalten sind, wobei es bevorzugt ist, wenn der Anteil dieser anionischen Tenside von 0,5 Gew.-% bis 8 Gew.-% bevorzugt von 2 Gew.-% bis 7,5 Gew.-% und insbesondere bevorzugt von 4 Gew.-% bis 7 Gew.-% bezogen auf das Gesamtgewicht der kosmetischen Zubereitung beträgt.

4. Reinigungszubereitung nach Anspruch 3 **dadurch gekennzeichnet, dass** keine nicht ionischen Tenside enthalten sind.

5. Reinigungszubereitung nach einem der vorhergehenden Ansprüche 1 bis 3 **dadurch gekennzeichnet, dass** als nichtionische Tenside Alkylglycoside, insbesondere Decylglycosid und/oder Cocoglycosid, enthalten sind.

6. Reinigungszubereitung nach Anspruch 5 **dadurch gekennzeichnet, dass** Decylglycosid und/oder Cocoglycosid enthalten sind, wobei der Anteil der Alkylglycoside von 0,5 Gew.-% bis 5 Gew.-% und bevorzugt von 2 Gew.-% bis 4 Gew.-%, bezogen auf das Gesamtgewicht der Reinigungszubereitung, beträgt.

7. Reinigungszubereitung nach einem der Ansprüche 5 oder 6 **dadurch gekennzeichnet, dass** keine anionischen Tenside enthalten sind.

8. Reinigungszubereitung nach einem der vorhergehenden Ansprüche **dadurch gekennzeichnet, dass** als amphotere und/oder zwitterionische Tenside Sodium Cocoamphoacetate, Disodium Cocoamphodiacetate, Sodium Cocoamphopropionate, Disodium Cocoamphodipropionate, Coco Betaine, Lauryl Betaine und/oder Cocamidopropylbetain enthalten sind.

9. Reinigungszubereitung nach einem der vorhergehenden Ansprüche **dadurch gekennzeichnet, dass** Cocamidopropylbetain in einem Anteil von 2,5 bis 7 Gew.-%, bevorzugt von 3 Gew.-% bis 6 Gew.-% und insbesondere bevorzugt von 3,5 Gew.-% bis 5,5 Gew.-%, bezogen auf das Gesamtgewicht der Reinigungszubereitung, enthalten ist, wobei es insbesondere bevorzugt, wenn neben Cocamidopropylbetain keine weiteren amphoteren und/oder zwitterionischen Tenside enthalten sind

10. Reinigungszubereitung nach einem der vorhergehenden Ansprüche **dadurch gekennzeichnet, dass** der Anteil von Caesalpinia Spinosa Gum von 0,15 bis 0,46 Gew.-% und bevorzugt von 0,2 bis 0,4 Gew.-%, bezogen auf das Gesamtgewicht der Zubereitung beträgt.

11. Reinigungszubereitung nach einem der vorhergehenden Ansprüche **dadurch gekennzeichnet, dass** zusätzlich Xanthan Gum enthalten ist, wobei es bevorzugt ist, wenn der Gesamtanteil von Xanthan Gum von 0,01 bis 0,3 Gew.-% bezogen auf das Gesamtgewicht der Reinigungszubereitung beträgt.

12. Reinigungszubereitung nach einem der Ansprüche 1 bis 10 **dadurch gekennzeichnet, dass** keine weiteren natürlichen polymeren Verdicker enthalten sind.

13. Reinigungszubereitung nach einem der Ansprüche 1-3 oder 5 bis 12 **dadurch gekennzeichnet, dass** die Zubereitung PEG-7 Glyceryl Cocoate enthält, wobei der Anteil von PEG-7 Glyceryl Cocoate vorteilhaft von 0,2 Gew.-% bis 5 Gew.-%, bevorzugt von 0,5 Gew.-% bis 2,5 Gew.-% und insbesondere bevorzugt von 1 Gew.-% bis 2 Gew.-%, bezogen auf das Gesamtgewicht der Reinigungszubereitung, beträgt.

14. Reinigungszubereitung nach einem der vorhergehenden Ansprüche **dadurch gekennzeichnet, dass** Natriumchlorid enthalten ist, wobei es vorteilhaft ist, wenn der Anteil von Natriumchlorid von 0,15 bis 1,2 Gew.-% und insbesondere vorteilhaft von 0,6 bis 1 Gew.-% bezogen auf das Gesamtgewicht der Zubereitung beträgt.

15. Reinigungszubereitung nach einem der Ansprüche 1-3 oder 5 bis 14 **dadurch gekennzeichnet, dass** PEG-200 Hydrogenated Glyceryl Palmate, PEG-40 Hydrogenated Castor Oil und/oder PEG-90 Glyceryl Isostearate enthalten sind, wobei es vorteilhaft ist, wenn der Anteil von PEG-200 Hydrogenated Glyceryl Palmate, PEG-40 Hydrogenated Castor Oil und/oder PEG-90 Glyceryl Isostearate von 0,1 Gew.-% bis 1,5 Gew.-%, bevorzugt von 0,2 Gew.-% bis 1,3 Gew.-%, insbesondere bevorzugt von 0,5 Gew.-% bis 1 Gew.-%, bezogen auf das Gesamtgewicht der Reinigungszubereitung, beträgt.

## Claims

1. Cosmetic cleansing preparation for use in the shower comprising, based on the total weight of the preparation,
a) 0.5 to 8% by weight anionic and/or non-ionic surfactants,
b) 2.5 to 7% by weight amphoteric and/or zwitterionic surfactants, and
c) Caesalpinia spinosa gum,
**characterized in that** no homopolymers or copolymers of acrylic acid are present and **in that** the proportion of Caesalpinia spinosa gum is from 0.1 to 1% by weight.

2. Cleansing preparation according to Claim 1, **characterized in that** one or more anionic surfactants are present according to the formula (I)
RO-(CH₂CH₂O)ₓ-(CH₂-CHR¹O)_{y}-(CH₂CH₂O)_{z}-SO₃⁻ M⁺ (I)
**characterized in that**
R is a linear or branched C₈-C₁₈-alkyl radical or mixtures of different linear or branched C₈-C₁₈-alkyl radicals;
R¹ is methyl, ethyl or mixtures thereof;
M+ is a cation, selected from the group consisting of alkali metals, NH₄⁺ and HNR²₃⁺, wherein R² is selected from the group consisting of linear or branched alkyl radicals, CH₂CH₂OH and CH₂CH(OH)CH₃;
x is an integer in the range of 0-3;
y is an integer in the range of 0-10; and
z is an integer in the range of 0-30.

3. Cleansing preparation according to either of the preceding claims, **characterized in that** sodium laureth sulfate and/or ammonium laureth sulfate are present, wherein it is preferred if the proportion of these anionic surfactants is from 0.5% by weight to 8% by weight, preferably from 2% by weight to 7.5% by weight and particularly preferably from 4% by weight to 7% by weight, based on the total weight of the cosmetic preparation.

4. Cleansing preparation according to Claim 3, **characterized in that** no non-ionic surfactants are present.

5. Cleansing preparation according to any of the preceding Claims 1 to 3, **characterized in that** alkyl glycosides, particularly decyl glycoside and/or coco glycoside, are present as non-ionic surfactants.

6. Cleansing preparation according to Claim 5, **characterized in that** decyl glycoside and/or coco glycoside are present, wherein the proportion of the alkyl glycosides is from 0.5% by weight to 5% by weight and preferably from 2% by weight to 4% by weight, based on the total weight of the cleansing preparation.

7. Cleansing preparation according to either of Claims 5 or 6, **characterized in that** no anionic surfactants are present.

8. Cleansing preparation according to any of the preceding claims, **characterized in that** the amphoteric and/or zwitterionic surfactants present are sodium cocoamphoacetate, disodium cocoamphodiacetate, sodium cocoamphopropionate, disodium cocoamphodipropionate, coco betaine, lauryl betaine and/or cocamidopropyl betaine.

9. Cleansing preparation according to any of the preceding claims, **characterized in that** cocamidopropyl betaine is present at a proportion of from 2.5 to 7% by weight, preferably from 3% by weight to 6% by weight and particularly preferably from 3.5% by weight to 5.5% by weight, based on the total weight of the cleansing preparation, wherein it is particularly preferred if no other amphoteric and/or zwitterionic surfactants are present besides cocamidopropyl betaine.

10. Cleansing preparation according to any of the preceding claims, **characterized in that** the proportion of Caesalpinia spinosa gum is from 0.15 to 0.46% by weight and preferably from 0.2 to 0.4% by weight, based on the total weight of the preparation.

11. Cleansing preparation according to any of the preceding claims, **characterized in that** xanthan gum is additionally present, wherein it is preferred if the total proportion of xanthan gum is from 0.01 to 0.3% by weight, based on the total weight of the cleansing preparation.

12. Cleansing preparation according to any of Claims 1 to 10, **characterized in that** no other natural polymeric thickeners are present.

13. Cleansing preparation according to any of Claims 1-3 or 5 to 12, **characterized in that** the preparation comprises PEG-7 glyceryl cocoate, wherein the proportion of PEG-7 glyceryl cocoate is advantageously from 0.2% by weight to 5% by weight, preferably from 0.5% by weight to 2.5% by weight and particularly preferably from 1% by weight to 2% by weight, based on the total weight of the cleansing preparation.

14. Cleansing preparation according to any of the preceding claims, **characterized in that** sodium chloride is present, wherein it is advantageous if the proportion of sodium chloride is from 0.15 to 1.2% by weight and particularly advantageously from 0.6 to 1% by weight, based on the total weight of the preparation.

15. Cleansing preparation according to any of Claims 1-3 or 5 to 14, **characterized in that** PEG-200 hydrogenated glyceryl palmate, PEG-40 hydrogenated castor oil and/or PEG-90 glyceryl isostearate are present, wherein it is advantageous if the proportion of PEG-200 hydrogenated glyceryl palmate, PEG-40 hydrogenated castor oil and/or PEG-90 glyceryl isostearate is from 0.1% by weight to 1.5% by weight, preferably from 0.2% by weight to 1.3% by weight, particularly preferably from 0.5% by weight to 1% by weight, based on the total weight of the cleansing preparation.

## Revendications

1. Préparation cosmétique nettoyante pour utilisation sous la douche comprenant, par rapport au poids total de la composition,
a) 0,5 à 8 % en poids de tensioactifs anioniques et/ou non ioniques,
b) 2,5 à 7 % en poids de tensioactifs amphotères et/ou zwitterioniques, et
c) de la Caesalpinia Spinosa Gum,
**caractérisée en ce qu'**elle ne contient aucun homo- ou copolymère de l'acide acrylique, et **en ce que** la proportion de Caesalpinia Spinosa Gum est de 0,1 à 1 % en poids.

2. Préparation nettoyante selon la revendication 1, **caractérisée en ce qu'**elle contient un ou plusieurs tensioactifs de Formule (I)
RO-(CH₂CH₂O)ₓ-(CH₂-CHR¹O)_{y}-(CH₂CH₂O)_{z}-SO₃⁻ M⁺ (1)
**caractérisée en ce que**
R représente un radical alkyle en C₈-C₁₈ linéaire ou ramifié ou des mélanges de différents radicaux alkyle en C₈-C₁₈ linéaires ou ramifiés ;
R¹ représente un méthyle, un éthyle ou des mélanges de ceux-ci ;
M⁺ représente un cation choisi dans le groupe consistant en les métaux alcalins, NH₄⁺ et HNR²₃⁺, R² étant choisi dans le groupe consistant en les radicaux alkyle linéaires ou ramifiés, CH₂CH₂OH et CH₂CH(OH)CH₃ ;
x représente un nombre entier dans la plage de 0 à 3 ;
y représente un nombre entier dans la plage de 0 à 10 ; et
z représente un nombre entier dans la plage de 0 à 30.

3. Préparation nettoyante selon l'une des revendications précédentes, **caractérisée en ce qu'**elle contient du laurethsulfate de sodium et/ou du laurethsulfate d'ammonium, auquel cas on préfère que la proportion de ces tensioactifs anioniques soit de 0,5 % en poids à 8 % en poids, de préférence de 2 % en poids à 7,5 % en poids et tout particulièrement de 4 % en poids à 7 % en poids par rapport au poids total de la préparation cosmétique.

4. Préparation nettoyante selon la revendication 3, **caractérisée en ce qu'**elle ne contient aucun tensioactif ionique.

5. Préparation nettoyante selon l'une des revendications précédentes 1 à 3, **caractérisée en ce qu'**elle contient comme tensioactifs anioniques des alkylglycosides, en particulier du décylglycoside et/ou du coco-glycoside.

6. Préparation nettoyante selon la revendication 5, **caractérisée en ce qu'**elle contient du décylglycoside et/ou du coco-glycoside, la proportion des alkylglycosides étant de 0,5 % en poids à 5 % en poids et de préférence de 2 % en poids à 4 % en poids par rapport au poids total de la préparation nettoyante.

7. Préparation nettoyante selon l'une des revendications 5 ou 6, **caractérisée en ce qu'**elle ne contient aucun tensioactif anionique.

8. Préparation nettoyante selon l'une des revendications précédentes, **caractérisée en ce qu'**elle contient comme tensioactifs amphotères et/ou zwitterioniques du cocoamphoacétate de sodium, du cocoamphodiacétate disodique, du cocoamphopropionate de sodium, du cocoamphodipropionate disodique, des cocobétaïnes, des laurylbétaïnes et/ou de la coco-amidopropylbétaïne.

9. Préparation nettoyante selon l'une des revendications précédentes, **caractérisée en ce qu'**elle contient la coco-amidopropylbétaïne en une quantité de 2,5 à 7 % en poids, de préférence de 3 % en poids à 6 % en poids et tout particulièrement de 3,5 % en poids à 5,5 % en poids par rapport au poids total de la préparation nettoyante, auquel cas on préfère en particulier qu'elle ne contienne, outre la coco-amidopropylbétaïne, aucun autre tensioactif amphotère et/ou zwitterionique.

10. Préparation nettoyante selon l'une des revendications précédentes, **caractérisée en ce que** la proportion de Caesalpinia Spinosa Gum est de 0,15 à 0,46 % en poids et de préférence de 0,2 à 0,4 % en poids par rapport au poids total de la préparation.

11. Préparation nettoyante selon l'une des revendications précédentes, **caractérisée en ce qu'**elle contient en outre de la gomme xanthane, auquel cas on préfère que la proportion totale de gomme xanthane soit de 0,01 à 0,3 % en poids par rapport au poids total de la préparation nettoyante.

12. Préparation nettoyante selon l'une des revendications 1 à 10, **caractérisée en ce qu'**elle ne contient aucun autre épaississant polymère naturel.

13. Préparation nettoyante selon l'une des revendications 1 à 3 ou 5 à 12, **caractérisée en ce que** la préparation contient du cocoate de glycéryle PEG-7, la proportion de cocoate de glycéryle PEG-7 étant de préférence de 0,2 % en poids à 5 % en poids, de préférence de 0,5 % en poids à 2,5 % en poids et tout particulièrement de 1 % en poids à 2 % en poids par rapport au poids total de la préparation nettoyante.

14. Préparation nettoyante selon l'une des revendications précédentes, **caractérisée en ce qu'**elle contient du chlorure de sodium, auquel cas il est avantageux que la proportion de chlorure de sodium soit de 0,15 à 1,2 % en poids et d'une manière particulièrement avantageuse de 0,6 à 1 % en poids par rapport au poids total de la préparation.

15. Préparation nettoyante selon l'une des revendications 1 à 3 ou 5 à 14, **caractérisée en ce qu'**elle contient du palmate de glycéryle hydrogéné PEG-200, de l'huile de ricin hydrogénée PEG-40 et/ou de l'isostéarate de glycéryle PEG-90, auquel cas il est avantageux que la proportion du palmate de glycéryle hydrogéné PEG-200, de l'huile de ricin hydrogénée PEG-40 et/ou de l'isostéarate de glycéryle PEG-90 soit de 0,1 % en poids à 1,5 % en poids, de préférence de 0,2 % en poids à 1,3 % en poids, d'une manière particulièrement préférée de 0,5 % en poids à 1 % en poids par rapport au poids total de la préparation nettoyante.
